# EUROPEAN PATENT SPECIFICATION

(11) **EP 2 804 559 B1**
(45) Date of publication and mention of the grant of the patent: **06.06.2018**
(21) Application number: 13705595.0
(22) Date of filing: 17.01.2013
(51) Int. Cl.: A61C 8/00, A61C 13/00, A61L 27/42

(54) **DENTAL OR BONE IMPLANT, IN PARTICULAR OF ALUMINA-ZIRCONIA NANOCOMPOSITE**
DENTAL- ODER KNOCHENIMPLANTAT, INSBESONDERE AUS EINEM ZIRKONIUMOXID-NANOKOMPOSIT
IMPLANT DENTAIRE OU OSSEUX CONSTITUÉ EN PARTICULIER D'UN NANOCOMPOSITE À BASE D'ALUMINE ET DE ZIRCONE

(30) Priority: 17.01.2012 IT TO20120029
(43) Date of publication of application: 26.11.2014
(73) Proprietor: Università Degli Studi Di Torino, 10124 Torino (IT); Consiglio Nazionale Delle Ricerche, 00185 Roma (IT)
(72) Inventor: COLUCCIA, Salvatore, 10138 Torino (IT); ALTRUDA, Fiorella, 10131 Torino (IT); CAROSSA, Stefano, 10128 Torino (IT); MARTRA, Gianmario, 10053 Bussoleno (Torino) (IT); MUSSANO, Federico, 10139 Torino (IT); TOLOSANO, Emanuela, 10147 Torino (IT); BELLOSI, Alida, 48018 Faenza (Ravenna) (IT); FAGA, Maria Giulia, 10125 Torino (IT)
(74) Representative: Vanzini, Christian
(86) International application number: PCT/IB2013/050425
(87) International publication number: WO 2013/108203

(56) References cited:
- US-A1- 2006 084 035
- US-A1- 2011 003 083
- US-A1- 2011 183 281

## Description

This invention relates in general to dental or more generally bone implants comprising a post and a thread made on the outer surface of the said post, through which the latter can be anchored to bone tissue.

A dental implant is a device designed to be surgically located within the mandibular bone or maxillary bone to support a dental prosthesis which will provide the patient with proper biofunctionality. The essential requirements for dental implants currently in use are biocompatibility, the ability to integrate with the bone, their lifetime and functional restoration of the organs (teeth) replaced, while possibly complying with aesthetic harmony.

The shape and components of dental implants are dictated by the mechanical characteristics of the basic material most commonly used for their manufacture, titanium, which together with its alloys accounts for approximately 97% of the market in this sector.

Generally pure titanium of grade 4 is used to make the fixture, that is the part of the implant within the bone, while the abutment, the part which will be covered by the dental prosthesis, and the connecting screw between the abutment and the fixture are made of grade 5 titanium. Titanium of grade 4 is considered to be pure (4 is an indicator of the presence of oxygen, carbon, iron and other impurities in residual percentages within the titanium matrix), while grade 5 titanium is an alloy comprising 6% by weight of aluminium and 4% by weight of vanadium plus other impurities as in grade 4 titanium.

The use of commercially pure titanium arises from the requirement to avoid the release of metal ions in contact with biological tissues, while ensuring the required mechanical properties. The use of grade 5 titanium is only desired by implant manufacturers to ensure a wide margin of mechanical safety for the implant system in relation to the action of chewing loads. The association between the build-up of metal such as aluminium and serious degenerative diseases such as Alzheimer's disease is of more than academic interest in this context, as the prosthetic components of implants are subject to the erosive action of the mouth's environment. Given the manifest need to control the bioavailability of potentially toxic elements, this requirement has been responded to, hitherto only experimentally, through containment barriers (e.g. surface coatings) or the choice of different materials. In this respect solid ceramic materials such as alumina (Al₂O₃) and zirconia (ZrO₂) have recently been proposed as an alternative to metal materials. Furthermore, from the biomechanical point of view the use of ceramic materials is particularly indicated to prevent force being transferred between the prosthesis and the surrounding biological tissue.

Successful development of a dental implant not only depends on the use of the right material, but also on its integration into the tissue in which it is placed. In fact a dental implant should not be regarded as a device that is merely capable of replacing natural teeth, but a system for rehabilitating the entire oral and mandibulatory apparatus. For the patient's quality of life it is therefore important that this rehabilitation should take place in the shortest time possible, and then persist for the longest time possible. A key aspect for the nature of integration is the application of anatomical, biomechanical, bioengineering and surface chemistry knowledge to the design and construction of devices.

Because of the particular mechanical stresses to which a dental implant must respond and the complexity of the physiological responses at the interface between the implant and tissues, the development of innovative devices having adequate structural characteristics capable of quickly and permanently integrating with the host tissue is now an aim of primary importance in the dental field. US2011/0003083 provides an example of this. In view of the requirements indicated above, an object of the invention comprises a process for the production of a dental or bone implant comprising the following steps:
providing a mixture of Al₂O₃ and ZrO₂ powders;
pressing the said mixture so as to obtain a green body;
processing the said green body so as to obtain a semi-finished product comprising a post and a thread provided on the outer surface of the said post, and
sintering the said semi-finished product,
wherein
after sintering the semi-finished product is subjected to bioactivation surface treatment with heat and pressure, keeping the said semi-finished product in contact with a solution of at least one compound selected from the group comprising oxyacids, hydrohalogen acids, alkaline metal bases and alkaline earth bases.

As will be appreciated, the implant according to the invention will find application not only in the dental field, but also in other medical sectors, for example orthopaedics.

Further characteristics and advantages of the implant according to the invention will be apparent from the following detailed description made with reference to the appended drawings provided purely by way of a non-limiting example, in which:
- Figure 1 is a graph showing the content by volume of m-ZrO₂ as a function of ageing time, in fired ATZ;
- Figure 2 is a graph showing the content by volume of m-ZrO₂ as a function of ageing time, in ATZ treated with H₃PO₄ for 4 hours;
- Figure 3 is a graph showing the Vickers microhardness (HV) of fired samples treated with acid, with different applied loads;
- Figure 4 is a graph showing the Vickers hardness of aged samples (fired ATZ) with different applied loads; and
- Figure 5 is a graph showing the Vickers hardness of samples aged for 4 hours treated with acid, with different applied loads.

A dental implant comprises a post, commonly known as the "fixture", which when in use constitutes the endosseous part of the implant, and a head portion, commonly known as the "abutment", which when in use is covered by the dental prosthesis. In the case of an implant for a different application, for example for orthopaedic use, this implant may be shaped differently according to requirements. The shape of the implant is not however essential for the purposes of the invention.

The post has a thread provided on its outer surface through which the device can be anchored into bony tissue.

According to the invention this implant is made of ceramic composite material based on Al₂O₃ and ZrO₂. Preferably this composite material is a nanocomposite.

For the purposes of this invention, by "nanocomposite" is meant a solid multiphase material in which the Al₂O₃ and ZrO₂ phases comprise grains having dimensions smaller than 100 nm.

The composite obtained has properties which are appreciably better than those of the components alone.

As far as this aspect is concerned, it must be borne in mind that oxide ceramics have good mechanical properties in terms of hardness and, consequently, resistance to wear, as well as established compatibility with biological systems. Pure alumina is in fact used in the biomedical field to make the heads of hip prostheses, while zirconia is not used for this purpose because despite greater toughness which makes it less brittle than alumina it tends to undergo a phase transition from tetragonal to monocline in vivo, with a consequent increase in volume and the initiation of fractures which result in breakdown of the prosthesis. Zirconia is however potentially more effective for biological applications because in addition to being tougher it has a potentially more reactive surface which makes it useful for the purposes of anchoring to bone.

Preferably the percentage by weight of ZrO₂ is 80% or above, the percentage by weight of Al₂O₃ being less than or equal to the 100-complement of the percentage by weight of ZrO₂.

The insert according to the invention is obtained by techniques that are conventional in the ceramics industry, such as linear pressing and isostatic cold pressing to obtain a green ceramic, and a subsequent sintering process. Before sintering the green body is processed by milling under digital control to obtain the desired profiles for the implant.

Preferably the implant so obtained undergoes surface heat treatment to optimise integration with bone tissue (bioactivation). In fact, in addition to their excellent mechanical properties, oxide ceramics tend to be bioinert. It has been established that following treatment with adequate concentrations of phosphoric acid solution under hydrothermal conditions implant surfaces have proved to be active in inducing the deposition of hydroxyapatite, according to the "Kokubo test" normally used to assess the osteointegration possibilities of a biomaterial. In addition to this it has been established that the abovementioned surface treatment does not adversely affect the mechanical properties of the oxide nanocomposites considered. Furthermore, ageing tests under conditions in which the chemical/physical phenomena which can occur under physiological conditions can be accelerated have indicated that after bioactivation treatment nanocomposites should maintain their structural properties unaltered for residence times in host tissue which would correspond to the normal life expectancy of patients, even young ones.

Another important aspect is that of biocompatibility, which has been established through tests of the survival and adhesion of human osteoblast and epithelial cells to the oxide nanocomposites used, including when treated for the purposes of bioactivation.

More generally, surface treatment of the implant in an autoclave may take place in the presence of solutions of oxyacids, hydrohalogen acids, alkaline and alkaline earth metal bases under temperature conditions over 50°C, for times of more than 15 minutes.

### Experiments

### 1. Preparation and characterisation of the ceramic material

High purity powders were used to produce the materials: a mixture of Al₂O₃ and ZrO₂ containing 16% by weight of ZrO₂ marketed by Taimei under the name Taimicron, and a mixture of Al₂O₃ and ZrO₂ containing 20% by weight of Al₂O₃ marketed by Tosoh under the name TZ-3Y20AB. Examples of green bodies were obtained by linear pressing at 80 MPa followed by cold isostatic pressing at 200 MPa. The best conditions for the sintering process were heating at 50°C/hour up to 700°C, residence at 700°C for 2 hours, heating at 100°C/hour to the sintering temperature of 1500°C and residence at that temperature for 2 hours. Thermal expansion coefficients were measured up to 1300°C; the densities of the sintered bodies were measured using Archimedes' method.

The following mechanical properties were also considered: 4-point bend strength on a test bar of 45×5×5 mm (at least five samples for each lot) for the materials after firing, and on 35×2,5×2 mm bars for materials which had undergone ageing and surface treatment; hardness using the Vickers point indentation method, with a load of 9.8 N for 10 seconds on at least five samples for each lot of the materials after firing; in the case of treated and aged materials micro-hardness was measured using an indentation tester (FISCHERSCOPE HM-2000) varying the load up to 0.5 N to determine surface hardness. Here again a Vickers indenter comprising a square base diamond pyramid was used. Toughness was measured using an indentation method with a Vickers point, a load of 98 N for 10 seconds, with at least five measurements on each material, and Young's modulus was measured by the resonance frequency method on at least three samples for each lot, of dimensions 43×5×2 mm, using an H&P gain and phase analyser. Bend strength and hardness were measured on samples after firing and after ageing and treatment.

The microstructure of the samples was analysed using a Zeiss EVO 50 scanning electron microscope provided with an energy dispersion spectroscopy analyser to detect elemental composition.

XPS analyses were also performed using a SPECS X-ray photoelectron spectrometer (Phoibos MCD 150). The source was Mg-Kα radiation (1253.6 eV). The X-ray source was excited by a 150 W electron beam (12 mA, 12.5 kV). The spot dimensions of the irradiated region were 7×20 mm. Photoelectron emissions from the sample were analysed at a take-off angle of 90° under UHV conditions. No load compensation was applied during acquisition. After acquisition binding energies were calibrated on the Al 2p signal of Al₂O₃ with a binding energy BE = 74 eV. The accuracy of the binding energies reported was estimated to be ±0.1 eV. The areas of the XPS peaks were measured after subtracting a background. Atomic ratios were calculated after standardisation using Scofield factors for element "X". The spectra were processed using the Casa XPS v2.3.13 software package (Casa Software Ltd.) and Origin 7.1 software (Origin Laboratory Corp.). Spectral resolution was performed using Gaussian-Lorenzian functions (70%/30%) and the FWHM for each peak was determined.

### 2. Treatment of samples

The samples were washed using ultrasound in acetone, ethanol and deionised water and then immersed in a solution of H₃PO₄ or NaOH solution in different concentrations at 80°-100°C for times of between 4 and 24 hours in an autoclave or at atmospheric pressure. When treatment was complete the samples were taken from the hydroxylated solution, washed in deionised water and then dried at ambient temperature.

### 3. Bioactivation

The bioactivity of untreated and treated samples was assessed by merging the samples in a modified simulated body fluid (1.5×SBF). The 1.5×SBF was prepared with an ion concentration of 1.5 times that of the SBF proposed by Kokubo et al., and its pH value was adjusted to 7.4 through the addition of HCl and tri-hydroxymethylaminomethane. Each sample was immersed in 40 ml of 1.5×SBF and held at a constant temperature of 36.5°C for 2 and 4 weeks. Observation of a layer having a Ca/P ratio typical of simulated apatite forms by SEM-EDS was used as a criterion for the bioactivity of the samples.

### 4. Ageing experiments

The ageing experiments were carried out in autoclaves at a temperature of 134°C and a pressure of 2 bars for increasing times up to 20 hours (1 hour in an autoclave is theoretically equal to 3-4 hours in vivo).

The samples were laid out on a grid in the autoclave so that they were not in contact with the water during ageing, but only subjected to an atmosphere of water vapour. The effect of hydrothermal treatment on the tetragonal-monocline transformation was assessed quantitatively by X-ray diffractometry. The samples were scanned with Cu-Kα radiation (45 kV-40 mA) at a Bragg angle varying from 26° to 34° using a PAN analytical X Pert PRO X-ray diffractometer. The fraction of monocline phase was calculated using the Garvie and Nicholson method.

Transformation of the zirconia phase was observed using an atomic force microscope (AM) and a scanning electron microscope (SEM). The same area of the sample was photographed before and after the ageing process. The AFM (Park System XE100) was observed in non-contact mode. The dimensions of the scanned area were 5×5 µm², with a scanning frequency of 0.4 Hz. Measurements were made on a sample which was smooth and treated with thermal etching in air. Measurements of average roughness (Rₐ) were made using images of five random areas on the sample.

### 5. Results

### 5.1 Microstructure

Both the materials Al₂O₃-16wt%ZrO₂ (alumina matrix reinforced with zirconia particles, ZTA) and ZrO₂-20wt% Al₂O₃ (zirconia matrix reinforced with alumina particles, ATZ) comprise sub-micrometre grains. In particular Al₂O₃ grains of 0.881 ± 0.297 µm and ZrO₂ grains of 0.317 ± 0.103 µm were present in ZTA, while in ATZ the dimensions of the alumina and zirconia grains were similar (0.469 ± 0.174 µm). In both cases the alumina and zirconia grains were uniformly distributed. In the case of ZTA the alumina grains were larger, probably because of the low percentage of zirconia, which has a pinning effect on alumina grains.

### 5.2 Physical and mechanical properties

The properties of the composites are summarised in Table 1, which also provides properties of the monolithic materials for comparison.

**Table 1**

| **Composition** | **Density (%)** | **Thermal expansion coefficient (m °C)** | **Hardness (GPa)** | **Toughness (MPa m^{1/2})** | **Bend strength (MPa)** | **Young's modulus (GPa)** |
|---|---|---|---|---|---|---|
| **Al₂O₃**-**16wt%ZrO₂** | 100 | 8.2 | 21.3 ±1.5 | 3.9 ±0.05 | 441±24 | 363±5 |
| **ZrO₂-20wt% Al₂O₃** | 99.9 | 10.0 | 15.3 ±0.9 | 7.1±0.1 | 633±127 | 245±9 |
| **Al₂O₃¹** | 99.9 | 7.8 | 24.0 ±1.6 | 3.0 ±0.1 | 229±34 | 362±4 |
| **ZrO₂²** | 100 | 11.0 | 14.5 ±0.4 | 6.0 ±0.3 | 615±93 | 206±4 |

| | | | | | | |
|---|---|---|---|---|---|---|
| 1- Al₂O₃ Taimei 2- ZrO₂ Tosoh, stabilised with 3 wt% of yttria | | | | | | |

In general it will be seen that the compounds offer a good compromise in terms of hardness and toughness in comparison with the monolithic material.

In particular the ATZ composite has a similar hardness to pure zirconia and greater toughness. This indicates that ATZ is more tolerant to fractures than zirconia. This is an advantage because pure zirconia has a problem of tending to fracture, despite its promising mechanical properties.

The toughness and bend strength of ZTA are considerably better than those of the monolithic material, even though it is harder. Toughness and bend strength are however poorer than that for the other composite, thus resulting in greater brittleness. A significant standard deviation was observed for ZTA, in particular for strength values, an indicator of the presence of defects.

### 5.3 Surface characterisation

Before chemical treatments the surfaces of the oxides were characterised by XPS. The results obtained for ZTA and ATZ samples reveal the presence of atoms of aluminium, zirconium, yttrium, oxygen and contaminants (in small quantities) on the surfaces.

The spectra were calibrated on the Al 2p signal of Al₂O₃ having a binding energy BE=74 eV. The zirconium peak showed a doublet with the Zr 3d_{5/2} component at 181.8 ±0.1 eV. The difference of 2.4 eV in binding energy between the Zr 3d_{5/2} and Zr 3d_{3/2} peaks is consistent with the binding energy of Zr4+ in pure zirconia. The Y 3d_{5/2} component of the yttrium doublet found at 157 ± 0.1 eV, with a difference of 2 eV from the Y 3d_{3/2} peak, is characteristic of the stabiliser for the tetragonal phase of ZrO₂ in YSZ.

The O 1s peak is the result of four contributions at 529.7, 530.8, 532.0 and 533.3 ± 0.1 eV corresponding to the O²⁻ in the zirconia matrix, the O²⁻ in the alumina matrix, hydroxyl groups due to solutions from the chemisorption of water, and the hydrated surface layer of the material respectively.

The quantitative proportions of aluminium, zirconium, yttrium and oxygen expected for the ZTA and ATZ samples were calculated considering the quantities of ZrO₂, Al₂O₃ and Y₂O₃ used in the mixture of precursors (see Table 2).

**Table 2**

| **Element** | **Atomic % in ZTA (calculated)** | **Atomic % in ZTA (XPS)** | **Atomic % in ATZ (calculated)** | **Atomic % in ATZ (XPS)** | **Atomic % in ATZ treated with H₃PO₄ for 4 hours (XPS)** |
|---|---|---|---|---|---|
| **Al** | **36.6** | 37.9 | **13.4** | 13.5 | 13.7 |
| **Zr** | **2.8** | 3.7 | **21.8** | 19.9 | 21.0 |
| **O** | **60.5** | 57.5 | **64.1** | 63.4 | 61.8 |
| **Y** | **0.1** | 0.9 | **0.7** | 3.2 | 3.5 |

The calculated values considering the stoichiometric ratio are very close to the experimental values. This demonstrates that the surface chemical composition of ZTA and ATZ is very close to that of the bulk. It will also be noted that the experimental percentage of Y is greater than expected, indicating that the Y atoms segregate out at the grain boundaries in Y-TZP.

### 5.4 Chemical treatments

Various chemical treatments with H₃PO₄ and NaOH at different concentrations and temperatures for different treatment times were considered for inducing bioactivity on the surfaces of both the composites. The ability of these treatments to induce the formation of apatite on surfaces was tested by immersion in SBF. As a reference, untreated and treated ZTA and ATZ materials were immersed in a solution of SBF for 2 and 4 weeks.

No apatite formation was observed in the case of ZTA, indicating that there is a threshold percentage of zirconia below which bioactivity is not induced.

Of the various treatments, only one (42% by weight H₃PO₄ solution, 80°C, 4 hours in an autoclave) resulted in the formation of simulated apatite in the case of ATZ after four weeks immersion in SBF, using both static and dynamic methods. The surface was almost completely covered with a layer of calcium phosphate, in which the ratio between Ca and P was 1.6, as was to be expected for forms of apatite. An acicular morphology was observed in each test (both the static and the dynamic). The apatite began to grow preferably on the zirconia grains, confirming that this oxide is more effective for the nucleation of apatite. In addition to this, this aspect explains why the composite containing a low percentage of zirconia did not have the same ability to encourage the formation of apatite. To check the effect of phosphoric acid on ATZ, XPS spectra were obtained on samples after treatment. The spectra were calibrated in the same way as for the samples after firing. No significant differences were found between treated ATZ and fired ATZ in terms of Zr/Al ratio. In addition to this no phosphorus peak was observed, indicating that no phosphate phase had formed. Deconvolution of the 0 1s peak revealed interesting differences between fired ATZ and ATZ treated with phosphoric acid (see Table 3).

**Table 3**

| **O1s components** | **ZTA** | **ATZ** | **ATZ 4h H₃PO₄** |
|---|---|---|---|
| %O₂- (ZrO₂) | 9.3% | 48.7% | 43.7% |
| %O₂- (Al₂O₃) | 65.2% | 27.2% | 23.4% |
| %OH | 19.2% | 20.0% | 24.6% |
| %H2O | 6.3% | 4.1% | 8.3% |

In the case of treated ATZ an increase in OH and the percentage of water were noted. The bioactivity observed in this sample must probably be correlated with an increase in OH species on the surface due to a base-acid reaction between zirconia and phosphoric acid. The fact that the same treatment did not result in a similar effect in a sample rich in alumina confirms that alumina grains are not effective in the nucleation of apatite.

It is also important to note that the same treatment in a beaker did not produce any effect on bioactivity after the same treatment time; this indicates that pressure has an important part to play in accelerating the kinetics of surface modification.

### 5.5 Effect of ageing on ATZ

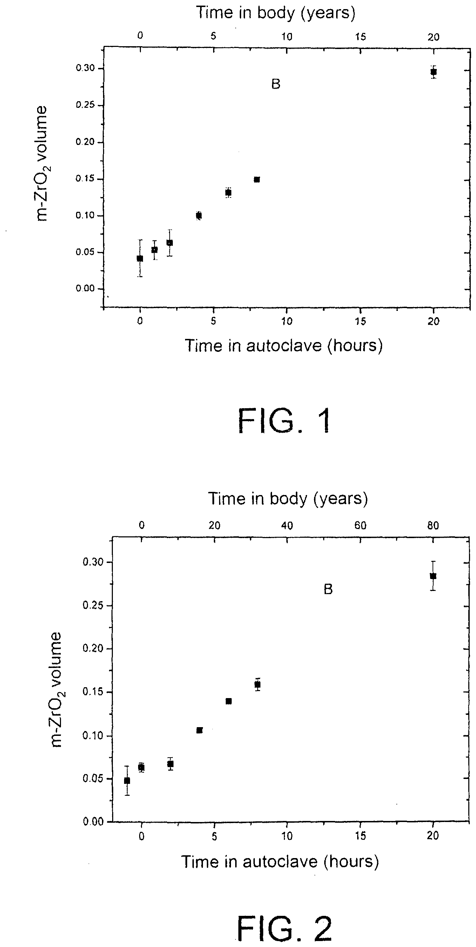

Various studies have demonstrated that when the percentage of ZrO₂ is below 22% by weight no ageing phenomena occur in similar composites, regardless of the grain size. The process of ageing on the ATZ composite was therefore studied to calculate the actual magnitude of the phase transition and its effect on mechanical properties to check the actual possibility of applying it in vivo. Figures 1 and 2 show the percentage by volume of the phase transition (calculated by the Garvie and Nicholson method) in relation to ageing time (up to 20 hours, corresponding to 80 years in vivo, a sufficiently long time for the service life of a prosthesis) for fired ATZ and ATZ treated with phosphoric acid. As in the case of the fired sample it can be seen that the quantity of monocline zirconia increases with ageing time and the correlation is almost linear. It will be seen that the phase transformation does not exceed a value of 28% by volume. Ageing did not reduce the strength of the ceramic after 20 hours and treatment with phosphoric acid in any event produced an increase in bend strength (see Table 4).

**Table 4**

| **Material** | **4 pt bend strength (MPa)** |
|---|---|
| **Fired ATZ** | 1075 ± 190 |
| **ATZ aged in an autoclave for 20 hours** | 1113 ± 142 |
| **ATZ aged in an autoclave for 90 hours** | 1002± 40 |
| **ATZ treated with phosphoric acid and aged in an autoclave for 20 hours** | 1246±124 |

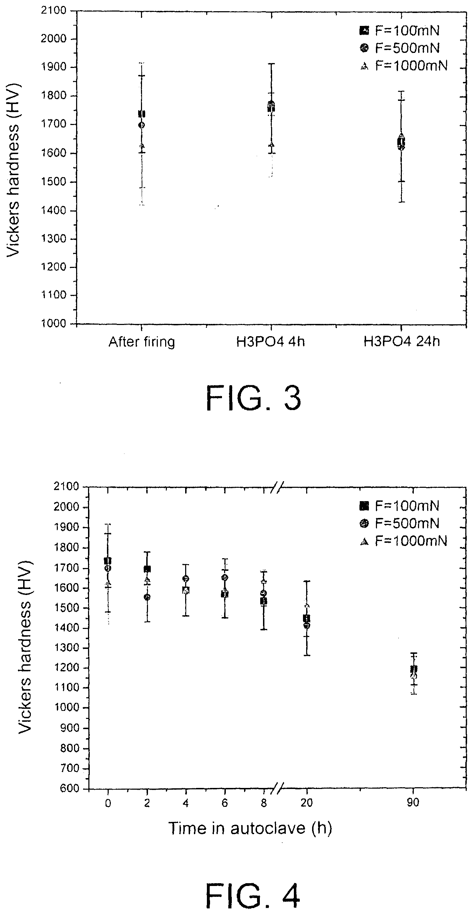

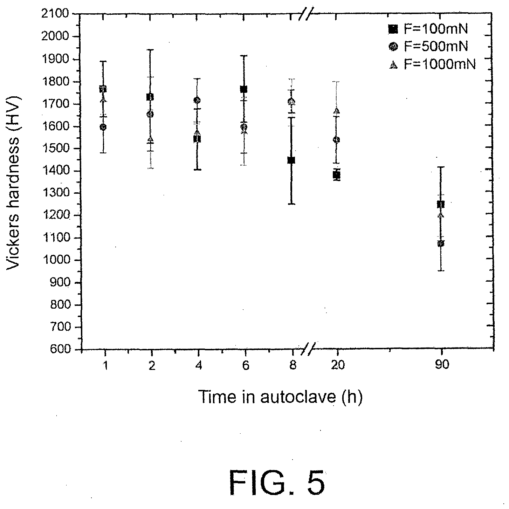

The effect of treatment and ageing on hardness is illustrated in Figure 3. Three different loads were used to determine any differences between the bulk and the surface; no such differences were however found. The main effect of ageing is a progressive decrease in hardness (Figure 4), but no trend was observed in the case of aged materials after treatment in phosphoric acid up to 20 hours. A significant fall can clearly be observed after 90 hours (Figure 5). SEM observations have shown that there is growth of micrograins on the surface of the zirconia grains with ageing, while AFM observations have demonstrated a phase transformation from tetragonal to monocline during ageing; these measurements have however shown that this transformation gives rise to a very small increase in mean roughness, which can explain their small influence on the mechanical properties of the material.

## Claims

1. A method for producing a dental or bone implant, comprising the following steps:
providing a mixture of powders of Al₂O₃ and ZrO₂;
pressing said mixture to obtain a green body;
machining said green body to obtain a semi-finished product comprising a post and a thread formed on an outer surface of the said post; and
subjecting said semi-finished product to sintering,
**characterized in that**
after sintering said semi-finished product is subjected to a bioactivation surface treatment with heat and pressure, including maintaining said semi-finished product in the presence of a solution of at least one compound selected from the group comprising oxyacids, hydrohalogen acids and alkali metal and alkaline earth metal bases.

2. A method according to Claim 1, in which the percentage by weight of ZrO₂ in said mixture of powders is greater than or equal to 80%, the percentage by weight of Al₂O₃ being less than or equal to the 100-complement of the percentage by weight of ZrO₂.

3. A method according to Claim 1 or 2, in which said bioactivation surface treatment further includes holding the said semi-finished product at a temperature above 50°C.

4. A method according to Claim 3, in which said bioactivation surface treatment includes holding said semi-finished product at a temperature above 50°C for longer than 15 minutes.

## Patentansprüche

1. Verfahren zum Herstellen eines Dental- oder Knochenimplantats, mit den folgenden Schritten:
Vorsehen einer Mischung von Pulvern von Al₂O₃ und ZrO₂;
Pressen besagter Mischung zum Erhalten eines Grünkörpers;
maschinelles Bearbeiten besagten Grünkörpers zum Erhalten eines halbfertigen Produkts mit einem Pfosten und einem Gewinde, das auf einer äußeren Oberfläche des besagten Postens ausgebildet ist; und
Unterziehen besagten halbfertigen Produkts einem Sintern,
**dadurch gekennzeichnet, dass**
nach dem Sintern besagtes halbfertiges Produkt einer Bioaktivierungsoberflächenbehandlung mit Hitze und Druck unterworfen wird, einschließlich eines Beibehaltens besagten halbfertigen Produkts in der Gegenwart einer Lösung mindestens einer Verbindung, die aus der Gruppe, die Oxysäuren, Halogenwasserstoffsäuren und Alkalimetall- und Erdalkalimetallbasen aufweist, ausgewählt wird.

2. Verfahren nach Anspruch 1, in dem der Prozentsatz nach Gewicht von ZrO₂ in besagter Mischung von Pulvern größer als oder gleich 80% ist, welcher Prozentsatz nach Gewicht von Al₂O₃ weniger als oder gleich dem 100-Komplement des Prozentsatzes nach Gewicht von ZrO₂ ist.

3. Verfahren nach Anspruch 1 oder 2, in dem besagte Bioaktivierungsoberflächenbehandlung ferner ein Halten des besagten halbfertigen Produkts bei einer Temperatur über 50°C aufweist.

4. Verfahren nach Anspruch 3, in dem besagte Bioaktivierungsoberflächenbehandlung ein Halten besagten halbfertigen Produkts bei einer Temperatur über 50°C für länger als 15 Minuten aufweist.

## Revendications

1. Procédé de production d'un implant dentaire ou osseux, comprenant les étapes suivantes :
fourniture d'un mélange de poudres d'Al₂O₃ et de ZrO₂ ;
compression dudit mélange pour obtenir un corps vert ;
usinage dudit corps vert pour obtenir un produit semi-fini comprenant un montant et un filetage formé sur une surface extérieure dudit montant ; et
soumission dudit produit semi-fini à un frittage,
**caractérisé en ce que**
après le frittage ledit produit semi-fini est soumis à un traitement de surface de bioactivation avec de la chaleur et de la pression, comprenant le maintien dudit produit semi-fini en présence d'une solution d'au moins un composé sélectionné dans le groupe comprenant des oxyacides, des acides hydrohalogénés et des bases de métaux alcalins et de métaux alcalino-terreux.

2. Procédé selon la revendication 1, dans lequel le pourcentage en poids de ZrO₂ dans ledit mélange de poudres est supérieur ou égal à 80 %, le pourcentage en poids d'Al₂O₃ étant inférieur ou égal au complément à 100 du pourcentage en poids de ZrO₂.

3. Procédé selon la revendication 1 ou 2, dans lequel ledit traitement de surface de bioactivation comprend en outre le maintien dudit produit semi-fini à une température supérieure à 50 °C.

4. Procédé selon la revendication 3, dans lequel ledit traitement de surface de bioactivation comprend le maintien dudit produit semi-fini à une température supérieure à 50 °C pendant plus longtemps que 15 minutes.
